(19) Europäisches Patentamt
European Patent Office
Office européen des brevets

(11) Veröffentlichungsnummer: **0 162 396**
**A2**

(12) # EUROPÄISCHE PATENTANMELDUNG

(21) Anmeldenummer: **85105840.4**

(22) Anmeldetag: **13.05.85**

(51) Int. Cl.[4]: **C 07 D 501/18**
C 07 D 501/46

(30) Priorität: **22.05.84 DE 3419014**

(43) Veröffentlichungstag der Anmeldung:
**27.11.85 Patentblatt 85/48**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Angerbauer, Rolf, Dr.**
**Sterntalerweg 29**
**D-5600 Wuppertal 1(DE)**

(54) **Verfahren zur Herstellung von 7-Amino-3-(3-formamidopyridinium) methyl-3-cephem-4-carboxylat und Verwendung zur Synthese von Beta-Lactamantibiotika.**

(57) Die Erfindung betrifft ein Verfahren zur Herstellung von 7-Amino-3-(3-formamidopyridinium) methyl-3-cephem-4-carboxylat der Formel (I)

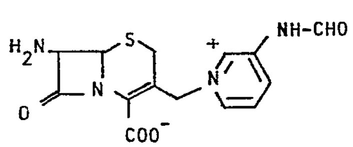

(I)

und seine Verwendung zur Synthese von ß-Lactamantibiotika der Formel (II)

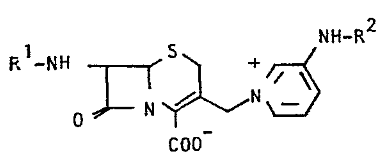

(II)

in denen
$R^1$ eine in der ß-Lactamchemie übliche seitenkette und
$R^2$ Formyl oder H
bedeutet.

EP 0 162 396 A2

0162396

BAYER AKTIENGESELLSCHAFT 5090 Leverkusen, Bayerwerk
Konzernverwaltung RP
Patentabteilung Ad/Lö-c

# Verfahren zur Herstellung von 7-Amino-3-(3-formamidopyridinium)methyl-3-cephem-4-carboxylat und Verwendung zur Synthese von ß-Lactamantibiotika

Die Erfindung betrifft ein Verfahren zur Herstellung von 7-Amino-3-(3-formamidopyridinium)methyl-3-cephem-4-carboxylat der Formel (I)

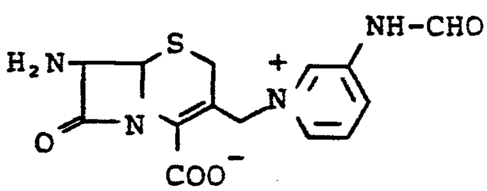

(I)

und seine Verwendung zur Synthese von ß-Lactamantibiotika der Formel (II)

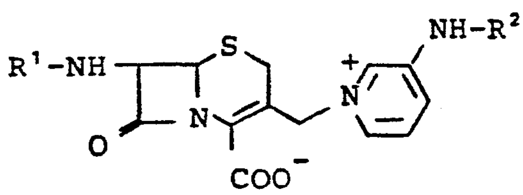

(II)

in denen

$R^1$ eine in der ß-Lactamchemie übliche Seitenkette

und

$R^2$ Formyl oder H

bedeutet.

Die Verbindung der Formel (I) wird hergestellt, indem man eine Verbindung der Formel (III),

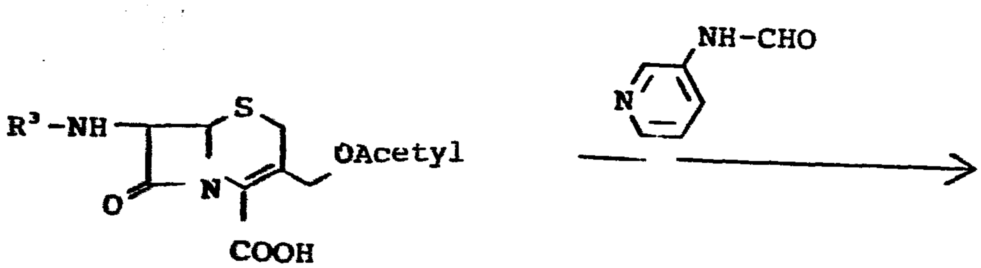

(III)

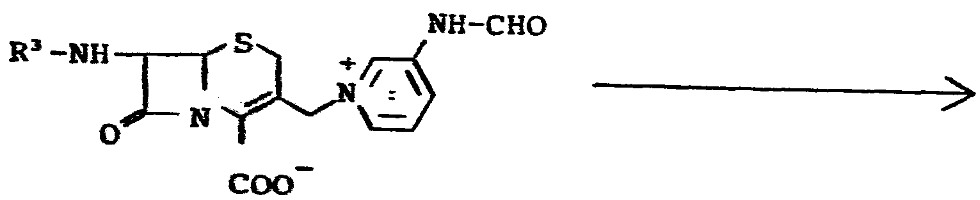 (I)

(IV)

in der $R^3$ eine Phenacetyl- oder eine Thienylacetylgruppe bedeutet, mit 3-Formamidopyridin zu einer Verbindung der Formel (IV) umsetzt, aus der man durch Abspaltung der Aminoschutzgruppe $R^3$ die Verbindung der Formel (I) erhält.

Die Umsetzung von Verbindungen der Formel III mit 3-Formamidopyridin zu Verbindungen der Formel IV erfolgt in organischen oder wäßrigen Lösungsmitteln, bei Temperaturen zwischen 20°C und 100°C, vorzugsweise zwischen 60°C und 80°C, unter Zugabe eines geeigneten Salzkatalysators.

Geeignete Salzkatalysatoren sind zum Beispiel NaBr, KJ, KSCN, NaSCN, LiJ.

Ein bevorzugtes organisches Lösungsmittel ist Dimethylformamid.

Besonders bevorzugt ist die Reaktion in wäßriger Lösung unter Verwendung eines großen Überschusses KSCN.

Es ist günstig, 3-Formamidopyridin in geringem Überschuß einzusetzen, wobei der Zusatz von 1 Äquivalent anorganischer Base, bevorzugt Natriumhydrogencarbonat, vorteilhaft ist.

Es ist vorteilhaft die Verbindungen der Formel (IV) nicht zu isolieren, sondern direkt nach Entsalzung mit einem Adsorberharz in die Verbindung der Formel (I) zu überführen. Dazu wird die bei der Reaktion (III) $\longrightarrow$ (IV) erhaltene Reaktionslösung mit Wasser verdünnt und dann mit einem Adsorberharz, bevorzugt HP 20, verrührt. Das Adsorberharz wird mit Wasser gewaschen und dann durch mehrmaliges Verrühren des Adsorberharzes mit einem Gemisch aus Wasser und einem organischen Lösungsmittel, bevorzugt mit einem Wasser/Acetonitril-Gemisch, bevorzugt im Verhältnis 20/80 bis 5/95, die Verbindung der Formel (IV) vom Harz eluiert. Nach Abziehen des organischen Lösungsmittels im Vakuum erhält man eine wäßrige Lösung der Verbindung der Formel (III).

Die Abspaltung der Aminschutzgruppe $R^3$ erfolgt enzymatisch mit immobilisierter Penicillin G Acylase bei pH 7-8, bevorzugt bei pH 7,8 direkt in der wäßrigen Lösung der Verbindung der Formel (IV). Während der enzymatischen Spaltung wird der pH-Wert durch Zugabe einer Base, wie z.B. Natriumhydoxid oder Triethylamin, konstant behalten.

Die Isolierung der Titelverbindung der Formel (I) erfolgt direkt aus der wäßrigen Spaltlösung durch Zugabe von konz. Salzsäure und einem organischen Lösungsmittel, bevorzugt Aceton, als kristallines Hydrochlorid.

Durch Kupplung mit Precursorsäuren lassen sich aus (I) Cephalosporine der Formel (II) ($R^2$=Formyl) herstellen, woraus man durch Abspaltung der Formylgruppe in bekannter Weise Verbindungen der Formel (II)($R^2$=H) erhält.

0162396

Beispiel 1

7-Amino-3-(3-formamidopyridinium)methyl-3-cephem-4-carboxylat

32 g 3-Acetoxymethyl-7ß-phenylacetamido-3-cephem-4-carbonsäure und 6,8 g Natriumhydrogencarbonat werden in 32 ml Wasser gelöst. Nach Zugabe von 144 g Kaliumrhodanid und 12,1 g 3-Formamidopyridin wird 4 h bei 65° gerührt. Die Reaktionslösung wird mit 96 ml Wasser verdünnt und dann mit 1 000 g Adsorberharz HP 20 verrührt. Das Adsorberharz wird abgesaugt und 3 x mit 600 ml Wasser gewaschen. Anschließend wird das Adsorberharz 3 x mit 600 ml Acetonitril/Wasser 95/5 verrührt und abgesaugt. Die Acetonitril/Wasser-Eluate werden vereinigt und das Acetonitril wird im Vakuum abgezogen. Die zurückbleibende wäßrige Lösung ( ≈ 500 ml) wird mit 4 N Triethylamin in Ethanol auf pH 7.8 gestellt und 16 g immobilisierte Penicillin G Acylase werden zugegeben. Der pH-Wert wird während der enzymatischen Spaltung konstant bei 7,8 gehalten. Nach Beendigung der Spaltung wird vom Enzymharz abfiltriert, mit konz. Salzsäure auf pH 2 gestellt und vom entstehenden Niederschlag über Kieselgur abgesaugt. Das Filtrat wird zu 2 l Aceton getropft, wobei das gewünschte Produkt als Hydrochlorid auskristallisiert.

Ausbeute: 13,7 g (x HCl x $H_2O$, 43 %).

$^1$H-NMR ($D_2O$)
$\delta$ (ppm) = 9.53 (1H, s, H-2-Py); 8.70 (1H, d, J=7 Hz, H-6-Py); 8.43 (2H, m, H-4-Py, CHO);

8.01 (1H, dd, J=8 Hz, J=7 Hz, H-5-Py); 5.65 (1H, d, J=14 Hz, $CH_2$-Py); 5.35 (1H, d, J=14 Hz, $CH_2$-Py); 5.30 (1H, d, J=5 Hz, H-7-Lactam); 5.19 (1H, d, J=5 Hz, H-6-Lactam); 3.71 (1H, d, J=18 Hz, S-$CH_2$).

Beispiel 2

7ß-[(Z)-2-(2-Aminothiazol-4-yl)-2-methoxyiminoacetamido]-3-(3-formamidopyridinium)methyl-3-cephem-4-carboxylat

710 mg (3.58 mmol) (Z)-2-(2-Aminothiazol-4-yl)-2-methoxyiminoessigsäure werden unter Stickstoff bei Raumtemperatur in 4.5 ml absol. Dimethylformamid gelöst. Nach Zugabe von 230 µl Tripropylamin und 310 µl Tributylamin wird auf -50°C gekühlt. 290 µl Mesylchlorid werden zugegeben, und die Lösung wird 30 Minuten schnell zu einer auf 0°C gekühlten Lösung von 963 mg (2.6 mmol) 7-Amino-3-(3-formamidopyridinium)methyl-3-cephem-4-carboxylat (x HCl) in 1.4 ml Wasser und 1.4 ml Triethylamin gegeben. Nach 5 Minuten wird die Reaktionslösung auf 400 ml Aceton gegossen. Der Niederschlag wird abgesaugt, getrocknet und über Adsorberharz HP 20 chromatographiert (Laufmittel: Acetonitril/Wasser 5/95).

Ausbeute: 700 mg (52 %).

$^1H$ (DMSO-$d_6$)
$\delta$ (ppm) = 9.67 (1H, s, H-2-Py); 9.58 (1H, d, J=9 Hz, NH); 9.24 (1H, d, J=7 Hz, H-6-Py); 8.74

(1H, d, J=8 Hz, H-4-Py); 8.54 (1H, s, CHO); 8.13 (1H, m, H-5-Py); 7.24 (2H, bs, $NH_2$); 6.72 (1H, s, Thiazol); 5.73 (1H, d, J=14 Hz, $CH_2$-Py); 5.68 (1H, dd, J=9 Hz, J=5 Hz, H-7-Lactam); 5.24 (1H, d, J=14 Hz, $CH_2$-Py); 5.09 (1H, d, J=5 Hz, H-6-Lactam); 3.80 (3H, s, $OCH_3$); 3.55 (1H, d, J=18 Hz, S-$CH_2$); 3.15 (1H, d, J=18 Hz, S-$CH_2$).

Beispiel 3

7ß-/(Z)-2-(2-Aminothiazol-4yl)-2-methoxyiminoacetamido/-3-(3-aminopyridinium)methyl-3-cephem-4-carboxylat

520 mg (1 mmol) 7ß-/(Z)-2-(2-Aminothiazol-4-yl)-2-methoxyiminoacetamido/-3-(3-formamidopyridinium)methyl-3-cephem-4-carboxylat werden in 6 ml Methanol suspendiert und durch Zugabe von 0.4 ml konz. Salzsäure in Lösung gebracht. Nach 5 h wird das Methanol im Vakuum abgezogen und der Rückstand in 20 ml Wasser aufgenommen. Mit Ionenaustauscher MP 62 wird neutralisiert und dann gefriergetrocknet.

Ausbeute: 350 mg (71 %).

$^1$H-NMR (DMSO-$d_6$)
$\delta$ (ppm) = 9.51 (1H, d, J=9 Hz, NH); 8.52 (1H, s, H-2-Py); 8.44 (1H, d, J=7 Hz, H-6-Py); 7.71 (1H, dd, J=7 Hz, J=8 Hz, H-5-Py); 7.63 (1H, d, J=8 Hz, H-4-Py); 7.26 (2H,

bs, $NH_2$); 6.83 (2H, bs, $NH_2$); 6.72 (1H, s, Thiazol); 5.62 (1H, dd, J=9 Hz, J=5 Hz, H-7-Lactam); 5.60 (1H, d, J=13 Hz, $CH_2$-Py); 5.09 (1H, d, J=5 Hz); 5.08 (1H, d, J=13 Hz, $CH_2$-Py); 3.81 (3H, s, $OCH_3$); 3.53 (1H, d, J=18 Hz, S-$CH_2$); 3.07 (1H, d, J=18 Hz, S-$CH_2$).

Patentansprüche

1. Verfahren zur Herstellung von 7-Amino-3-(3-formamidopyridinium)methyl-3-cephem-4-carboxylat der Formel (I)

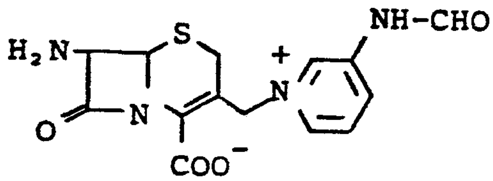

(I)

dadurch gekennzeichnet, daß man eine Verbindung der Formel (III)

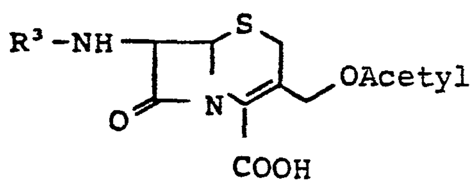

(III)

mit 3-Formamidopyridin zur Verbindung der Formel (IV)

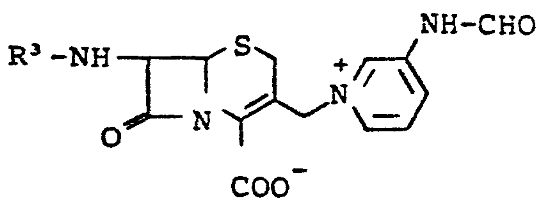

(IV)

umsetzt, worin $R^3$ eine Phenacetyl- oder eine Thienylacetylgruppe bedeutet und die Verbindung der Formel (IV), gegebenenfalls ohne vorausgehende Isolierung, direkt nach Entsalzung mit einem Adsorberharz in die Verbindung der Formel (I) überführt.

2. Verfahren nach Anspruch 1, dadurch gekennzeichnet, daß man die Umsetzung von (III) zu (IV) in einem organischen oder wäßrigen Lösungsmittel durchführt.

3. Verfahren nach Anspruch 2 durchgeführt in Gegenwart eines Salzkatalysators wie NaBr, KJ, KSCN, NaSCN oder LiJ.

4. Verfahren nach Anspruch 1, 2 oder 3, dadurch gekennzeichnet, daß man das 3-Formamidopyridin in geringem Überschuß einsetzt.

5. Verfahren nach Anspruch 1, 2, 3 oder 4, dadurch gekennzeichnet, daß man die Aminoschutzgruppe $R^3$ enzymatisch abspaltet und (I) aus der Spaltlösung isoliert.

6. Verwendung von Verbindungen der Formel (I)

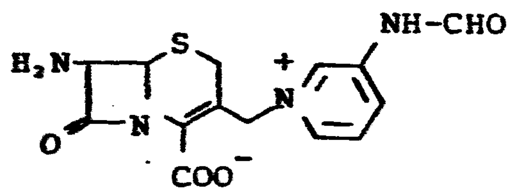

(I)

zur Synthese von ß-Lactamantibiotika der Formel (II)

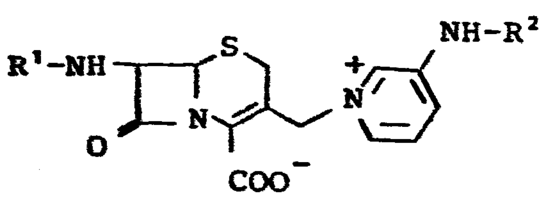

(II)

in denen

$R^1$ eine in der ß-Lactamchemie übliche Seitenkette

und

$R^2$ Formyl oder H

bedeutet.

7. 7-Amino-3-(3-formamidopyridinium)methyl-3-cephem-4-carboxylsäure, deren Anion und deren Salze.